(19) Europäisches Patentamt
European Patent Office
Office européen des brevets

(11) Veröffentlichungsnummer: **0 051 852**
**B1**

# (12) EUROPÄISCHE PATENTSCHRIFT

(45) Veröffentlichungstag der Patentschrift: **24.07.85**

(51) Int. Cl.⁴: **C 07 D 513/04,** A 61 K 31/54, A 61 K 31/18, A 61 K 31/505 // (C07D513/04, 277:00, 239:00)

(21) Anmeldenummer: **81109501.7**

(22) Anmeldetag: **04.11.81**

(54) **Pyrimido(4,5b)(1,4)thiazine und Verfahren zu ihrer Herstellung.**

(30) Priorität: **08.11.80 DE 3042187**

(43) Veröffentlichungstag der Anmeldung: **19.05.82 Patentblatt 82/20**

(45) Bekanntmachung des Hinweises auf die Patenterteilung: **24.07.85 Patentblatt 85/30**

(84) Benannte Vertragsstaaten: **FR GB IT NL SE**

(56) Entgegenhaltungen: **FR - A - 2 181 827**

(73) Patentinhaber: **Stiftung Forschungsinstitut Borstel, Parkallee 1-40, D-2061 Borstel (DE)**

(72) Erfinder: **Seydel, Joachim Karl, Prof. Dr., Mülch 2, D-2061 Borstel (DE)**
Erfinder: **Visser, Klaus, Parkallee, D-2061 Borstel (DE)**

EP 0 051 852 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Verbindungen sowie Kombinationen mit diesen Verbindungen und ihre Herstellung beispielsweise zur Behandlung von Mikrobeninfektionen.

Die neuen Verbindungen haben die allgemeine Formel (I) – Fig. 1 der Zeichnung – oder eine tautomere Form derselben, worin R eine Amino- oder Hydroxygruppe und die Reste $R_1$ und $R_2$ gleich oder verschieden und jeder dieser beiden Reste unabhängig voneinander eine Alkylgruppe mit 1 bis 3 Kohlenstoffatomen oder Wasserstoff ist.

Es wurde gefunden, dass die neuen Verbindungen bzw. ihre Derivate oder Salze als Antagonisten des mikrobiellen Metabolismus vorteilhaft brauchbar sind.

Von der aus der französischen Auslegeschrift FR-A-2 181 827 bekannten Formel unterscheidet sich die Erfindung durch den andersartigen Substituenten $R_3$, wodurch eine andere therapeutische Wirkung erzielt wird, nämlich Einwirkung auf Mikroorganismen.

Die neuen Verbindungen der Formel (I) können nach der vorliegenden Erfindung wie folgt hergestellt werden (siehe Formelschema nach Figur 1): Man setzt eine Verbindung der Formel (II) mit einer Verbindung der Formel (III) um, wobei Hal ein Halogen wie Chlor, Brom oder Jod bedeutet und R eine Amino- oder Hydroxygruppe und die Reste $R_1$ und $R_2$ gleich oder verschieden, und jeder dieser beiden Reste unabhängig voneinander eine Alkylgruppe mit 1 bis 3 Kohlenstoffatomen oder Wasserstoff ist, und isoliert die so gebildete Verbindung.

In diesem Verfahren unterwirft man eine Verbindung des Typs (II) einer Cyclisierungsreaktion mit einem Halogenhydroxyacetonderivat (III), wobei R, $R_1$, $R_2$ und Hal die vorgenannten Bedeutungen haben. Verbindungen der Formeln (II) und (III) können mittels bekannter Standardreaktionen hergestellt werden.

Die neuen Verbindungen und ihre Salze inhibieren eines der Enzyme, die bei einer Biosynthese der Dihydrofolsäure von Bedeutung und daher für das Wachstum von Mikroorganismen, z.B. Bakterien, essentiell sind. Die neuen Verbindungen können deshalb in pharmakologischen Untersuchungen in vitro, in klinischen und diagnostischen Untersuchungen, die beispielsweise die Eigenschaften von Bakterien betreffen, verwendet werden. Wenn sie als Bakteriostatika verwendet werden, verursachen die Verbindungen eine Reduktion der Vermehrung von Mikroorganismen bei einer Konzentration von 100 µmol/l.

Es wurde ausserdem gefunden, dass diese Verbindungen synergistisch sowohl mit Sulfonamiden als auch mit Dihydrofolsäurereduktasehemmern vom Typ des 2,4-Diamino-5-(3′,4′,5′-trimethoxybenzyl)-pyrimidins (TMP) sowie deren Kombination wirken können. Die starke wechselseitige Potenzierung der antibakteriellen Wirkungen ist Folge einer sequentiellen Blockade der Folsäurebiosynthese. Der Bereich der durch solche Kombinationen ausgeübten antimikrobiellen Wirkung ist beträchtlich grösser als er aus der Aktivität von einer der Drogen zu erwarten war.

Organismen, die nur gering gegen die einzelnen Mittel empfindlich sind, werden bzw. sind gegenüber diesen Kombinationen hoch empfindlich. Die Resistenzentwicklung wird deutlich verzögert. Die Mikrobeninfektionen, gegen die diese Kombinationen wirksam sind, sind Infektionen, die von solchen Bakterien verursacht werden, die wenigstens einen wesentlichen Teil ihres Tetrahydrofolat-Kofaktorbedarfs bilden. Im besonderen sind diese infizierten Mikroorganismen solche, die ausreichend die hier beschriebenen Kombinationen absorbieren und in denen diese Kombinationen eine synergistische Wirkung in der Weise ausüben, dass sie die «de novo»-Synthese der benötigten Tetrahydrofolat-Kofaktoren beeinträchtigen bzw. unterbinden.

Es wurde im besonderen gefunden, dass, wenn Verbindungen der Formel (I) – Fig. 1 – mit einer Menge eines Sulfonamids und/oder eines Hemmstoffs vom Typ des 2,4-Diamino-5-(3′,4′,5′-trimethoxybenzyl)-pyrimidins (TMP) kombiniert werden, die als solche gewöhnlich nicht ausreichend ist, als antimikrobielles Mittel wirksam zu sein, die Kombination einer Verbindung der Formel (I) mit dieser normalerweise nicht wirksamen Menge des Sulfonamids und/oder des Hemmstoffs vom Typ des genannten Pyrimidins (TMP) eine Mischung bildet, die als wirksames Mittel gegen Mikroben wirkt. Dies ist besonders dann von Bedeutung, wenn die Menge der Verbindung der Formel (I) so gering ist, dass sie im wesentlichen bei der gegebenen Menge keine Mikrobenwirkung hat, aber in der Kombination eine Potenzierung eintritt, die in einigen Fällen sehr stark ist. Es ist daher unter Verwendung einer wirksamen potenzierenden Menge einer Verbindung der Formel (I) zusammen mit dem Sulfonamid und/oder dem DHF-Reduktase-Hemmstoff vom Typ des erwähnten Pyrimidins (TMP) möglich, die Menge des Sulfonamids und/oder des DHF-Reduktase-Hemmstoffs vom Typ des erwähnten Pyrimidins (TMP), die zur Inhibierung des Wachstums dieser Bakterien erforderlich ist, wesentlich zu verringern. Entsprechend den vorausgegangenen Ausführungen bedeutet die Bezeichnung «eine wirksame Menge», soweit sie in Verbindung mit den Bezeichnungen «DHF-Reduktase-Hemmstoff vom Typ des 2,4-Diamino-5-(3′,4′,5′-trimethoxybenzyl)-pyrimidins (TMP)» und «Sulfonamid» verwendet wird, entweder (a) eine Menge des DHF-Reduktasehemmers vom Typ des 2,4-Diamino-5-(3′,4′,5′-trimethoxybenzyl)-pyrimidins (TMP) oder Sulfonamids, die zu einem gewissen Grad als Mittel gegen Mikroben wirksam ist, die aber durch die Verwendung einer Verwendung einer Verbindung der Formel (I) potenziert wird, oder (b) eine Menge DHF-Reduktase-Hemmstoff vom Typ des 2,4-Diamino-5-(3′,4′,5′-trimethoxybenzyl)-pyrimidins (TMP) oder Sulfonamid, die als Mittel gegen Mikroben nicht wirksam ist, die aber, wenn sie mit einer Verbindung der Formel (I) kombiniert wird, eine Zubereitung bildet, dei ein wirksames Mittel gegen Mikroben ist.

Unter einer «wirksamen potenzierenden Menge» ist eine Menge der Verbindung der Formel (I)-Fig. 1 - zu verstehen, die in Wirksamkeit eines DHF-Reduktase-Hemmstoffs vom Typ des 2,4-Diamino-5-(3′,4′,5′-trimethoxybenzyl)-pyrimidin und/oder Sulfonamids so erhöht, daß eine verbesserte oder ausreichende Wirksamkeit für die gesamte Kombination erreicht wird.

Die Verhältnisse, in denen die antimikrobiell wirksamen Verbindungen der Formel (I) in den hier beschriebenen Kombinationen verwendet werden, können in weiten Grenzen variieren. Abhängig von der Natur und den Umständen der Verwendung, können Mischungen der Verbindungen der Formel (I) zusammen mit dem Sulfonamid und/oder dem DHF-Reduktase-Hemmstoff vom Typ des erwähnten Pyrimidins mit entsprechenden Anteilen und Dosierungen gewählt werden.

Die potenzierende Wirkung der Verbindungen der Formel (I) kann in vitro relativ leicht durch den Röhrchenverdünnungstest (MHK) und/oder bakterienkinetische Untersuchungen gezeigt werden.

Im folgenden wird ein Ausführungsbeispiel der Erfindung beschrieben:

2-Amino-4-hydroxy-5-nitro-6-mercaptopyrimidin (II; R = OH) (1,59 g ≙ 10 mmol) wurde bei Raumtemperatur unter Stickstoff in 100 ml wasserfreiem Methanol, welches 440 mg Natriumhydroxyd (≙ 11 mmol) enthielt, unter Rühren gelöst. Zur resultierenden hellgelben Lösung wurde eine Lösung von 1-hydroxy-3-jodpropanon-(2) (III; $R_1$ = $R_2$ = H, Hal = J) (2,2 g ≙ 11 mmol) in 100 ml Methanol während einer Stunde hinzugetropft und die erhaltene Mischung zwei weitere Stunden unter Stickstoff gerührt. Dann wurde vom Ausgefallenen abfiltriert, das Filtrat mit einem halben Tropfen Eisessig neutralisiert und das Lösungsmittel im Vakuum weitgehend entfernt. Die ausgefallene gelbe Substanz wurde abfiltriert, mit Methanol gewaschen und im Vakuum über Phosphorpentoxyd getrocknet. Die Ausbeute des so erhaltenen neuen Stoffs 2-Amino-4-hydroxy-6-hydroxymethyl-7H-pyrimido[4,5b][1,4]thiazin (I; R = OH, $R_1$ = $R_2$ = H) betrug 1,1 g ≙ 51,9%, der Schmelzpunkt 300 °C.

Weitere erfindungsgemässe Verbindungen können analog zum beschriebenen Verfahren hergestellt werden.

Potentielle Folsäureantagonisten der Formel (I) und Kombinationen derselben mit Sulfonamiden und/oder DHF-Reduktase-Hemmstoffen vom Typ des 2,4-Diamino-5-(3′,4′,5′-trimethoxybenzyl)-pyrimidins (TMP) können auf ihre eigene und in Kombination potenzierte antimikrobielle Wirkung mit Hilfe der Bakterienvermehrungskinetik wie folgt geprüft werden:

Es werden zu Bakterienkulturen (Beispiel E. coli) in der logarithmischen Wachstumsphase die genannten Inhibitoren allein oder in den oben beschriebenen Kombinationen hinzugefügt. Die Veränderung der Zahl der Bakterien als Funktion der Zeit wird mit einem Coulter Counter (Gesamtkeimzahl) bzw. mit Hilfe der Kolonienzählung (Plattentest, Lebendkeimzahl) ermittelt. Die Vermehrungskurven werden bei Abwesenheit und bei Anwesenheit der Hemmstoffe und ihrer oben beschriebenen Kombinationen ermittelt. Die aus ihnen ermittelten Änderungen in den Vermehrungsgeschwindigkeiten sind ein Mass für die antibakterielle Wirkung.

Die Figur 2 der Zeichnung zeigt:

Typische Vermehrungskurven von E. coli (mutaflor) (Temperatur: 37 °C, Nährflüssigkeit: modifizierter Sauton) in Gegenwart von 2-Amino-4-hydroxy-6-hydroxymethyl-7H-pyrimido[4,5b]-[1,4]thiazin (I, R = OH, $R_1$ = $R_2$ = H) (PTZ), Sulphisoxazol (SMZ) und Trimethoprim (TMP) allein und in Kombinationen wie oben beschrieben. TMP wurde 1,5 Stunden später als die anderen Verbindungen der Kultur zugesetzt. Die Kurven (Symbol), Hemmstoffkonzentrationen (μmol/l) und beobachtete Vermehrungsgeschwindigkeitskonstanten $k_{beob}$. ($10^4$ $sec^{-1}$) waren:

1. Coulter Counter (Gesamtkeimzahl) (x) 0,0 SMZ, 0,0 PTZ, 0,0 TMP, 3,94; (●) 2,0 SMZ, 0,0 PTZ, 0,0 TMP, 1,06; (o) 0,0 SMZ, 75,0 PTZ, 0,0 TMP, 3,82; (Δ) 0,0 SMZ, 0,0 PTZ, 0,4 TMP, 0,72; (▲) 2,0 SMZ, 75,0 PTZ, 0,0 TMP, 0,58; (◇) 2,0 SMZ, 0,0 PTZ, 0,4 TMP, 0,0; (▽) 0,0 SMZ, 75,0 PTZ, 0,4 TMP, 0,12; (▼) 2,0 SMZ, 75,0 PTZ, 0,4 TMP, 0,0.

2. Plattentest (Lebendkeimzahl) (◇) 2,0 SMZ, 0,0 PTZ, 0,4 TMP, – 0,67; (■) 0,0 SMZ, 75,0 PTZ, 0,4 TMP, −0,11; (◆) 2,0 SMZ, 75,0 PTZ, 0,4 TMP, −0,43.

**Patentansprüche**

1. Verbindungen der Formel (I) oder eine tautomere Form derselben

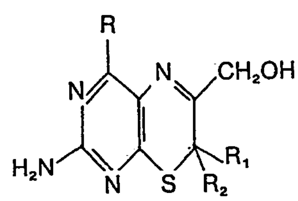

worin R eine Amino- oder Hydroxygruppe und die Reste $R_1$ und $R_2$ gleich oder verschieden, und jeder dieser Reste unabhängig voneinander eine Alkylgruppe mit 1 bis 3 Kohlenstoffatomen oder Wasserstoff ist.

2. Verfahren zur Herstellung einer Verbindung der Formel (I) oder eines Salzes davon, dadurch gekennzeichnet, dass man eine Verbindung der Formel (II) umsetzt mit einer Verbindung der Formel (III),

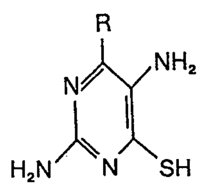

(II)

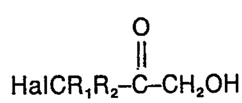

(III)

wobei Hal ein Halogen wie Chlor, Brom oder Jod bedeutet und die Reste R, $R_1$ und $R_2$ die in Anspruch 1 definierte Bedeutung haben, und die so gebildete Verbindung (I) isoliert.

3. Gemische mit Sulfonamiden und/oder Dihydrofolsäurereduktasehemmstoffen vom Typ des 2-4-Diamino-5-(3′,4′,5′-trimethoxybenzyl)-pyrimidins, in denen die Verbindung der Formel (I) gemäss Anspruch 1 enthalten ist, zur Anwendung als Bakteriostatika.

**Claims**

1. Compounds of the structural formula (I) or a tautomeric form thereof

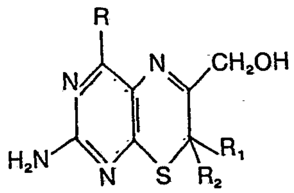

where R is an amino or hydroxyl group and where the rests $R_1$ and $R_2$ are similar or different and where each of these rests, independent from each other, is an alkyl group with 1 to 3 carbon atoms or hydrogen.

2. Procedure for the preparation of a compound with the structural formula (I) or a salt of such compound, characterized by the reaction of a compound with the structural formula (II) with a compound of the structural formula (III)

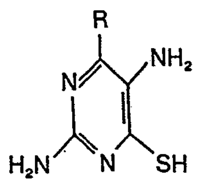

(II)

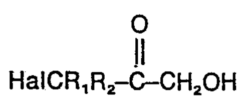

(III)

where Hal is a halogen as chlorine, bromine or iodine and where the rests R, $R_1$ and $R_2$ have the meaning as defined under claim 1 and further characterized by the isolation of the in this way formed compound (I).

3. Mixtures with sulphonamides and/or dihydrofolic acid reductase inhibitors of the type of 2,4-diamino-5-(3′,4′,5′-trimethoxybenzyl)-pyrimidine in which the compound of the structural formula (I) according to claim 1 is contained, for use as bacteriostatics.

**Revendications**

1. Composés de la formule (I) ou une forme tautomère de ceux-ci

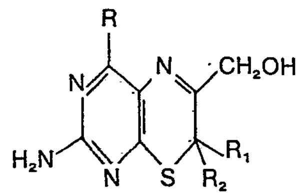

dans lesquels R est un groupe aminé ou hydroxyde et les restes $R_1$ et $R_2$, semblables ou dissemblables, étant chacun indépendamment de l'autre un groupe alkyle avec 1 à 3 atomes de carbone ou de l'hydrogène.

2. Procédé de fabrication d'un composé de la formule (I) ou d'un sel de celle-ci, caractérisé par le fait qu'on transforme un composé de la formule (II) avec un composé de la formule (III),

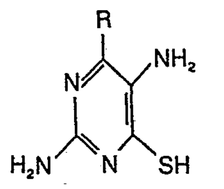

(II)

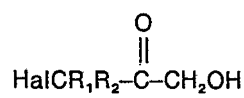

(III)

Hal signifiant un corps halogène tel que chlore, brome ou iode et les restes R, $R_1$ et $R_2$ ayant la signification définie dans la demande 1, et qu'on isole le composé (I) ainsi formé.

3. Mélanges avec des sulfamides et/ou des inhibiteurs de réductase de dihydroacide folique du type de la pyrimidine 2,4-Diamino-5-(3′,4′,5′-triméthoxybenzyle), dans lesquels est contenu le composé de la formule (I) d'après la demande 1, pour l'utilisation en tant que bactériostatique.

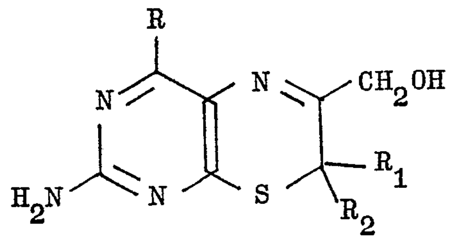

(I)

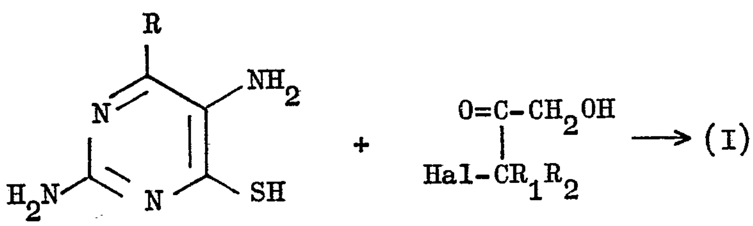

(II) (III)

Figur 1

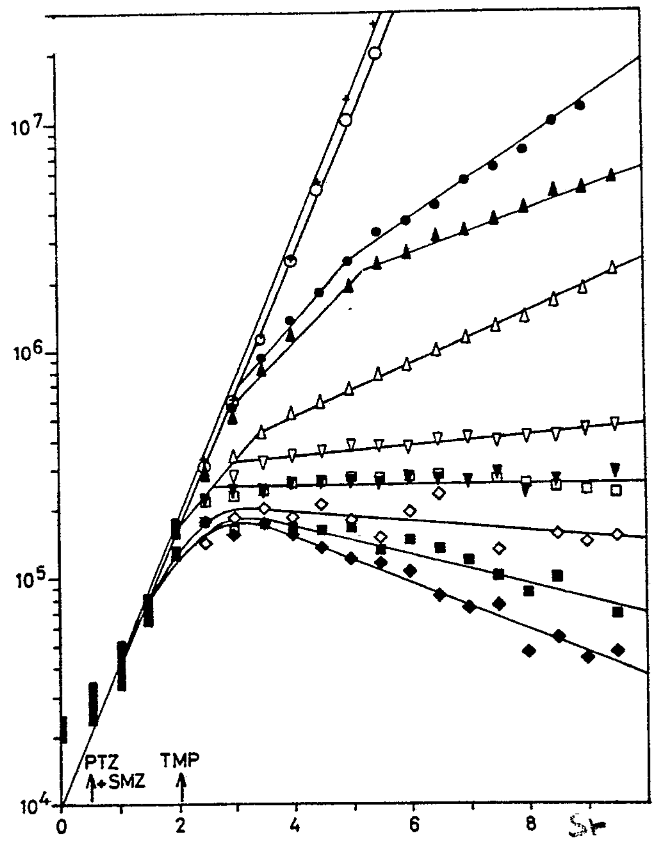


Figur 2